(19) Europäisches Patentamt / European Patent Office / Office européen des brevets

(11) **EP 4 542 225 A1**

(12) **EUROPEAN PATENT APPLICATION**
published in accordance with Art. 153(4) EPC

(43) Date of publication:
**23.04.2025 Bulletin 2025/17**

(51) International Patent Classification (IPC):
**G01N 33/487** (2006.01)   **G01N 30/72** (2006.01)
**G06N 3/08** (2023.01)

(21) Application number: 23827372.6

(52) Cooperative Patent Classification (CPC):
**G01N 30/72; G01N 33/487; G06N 3/08**

(22) Date of filing: **10.05.2023**

(86) International application number:
**PCT/KR2023/006352**

(87) International publication number:
**WO 2023/249252 (28.12.2023 Gazette 2023/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.06.2022 KR 20220074949**

(71) Applicant: **Innobation Bio Co., Ltd.**
**Seoul 03929 (KR)**

(72) Inventors:
• **KIM, Seung Ku**
  **Yongin-si Gyeonggi-do 16865 (KR)**
• **KIM, Ki Tae**
  **Seoul 06148 (KR)**

(74) Representative: **J A Kemp LLP**
**80 Turnmill Street**
**London EC1M 5QU (GB)**

(54) **BIOMARKERS FOR LUNG CANCER DIAGNOSIS AND METHOD FOR PROVIDING ARTIFICIAL INTELLIGENCE-BASED INFORMATION ON LUNG CANCER DIAGNOSIS**

(57) The present invention relates to a biomarker for diagnosing lung cancer and methods for providing artificial intelligence-based information for diagnosing lung cancer, and more particularly to a biomarker for diagnosing lung cancer, comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), a biomarker composition for diagnosing lung cancer, and a method for providing artificial intelligence-based information for diagnosing lung cancer using the biomarker.

As a result of establishing an artificial intelligence-based algorithm model for lung cancer diagnosis using the biomarkers for lung cancer diagnosis selected in the present invention, it was confirmed that the ability to early screen lung cancer is 90.21% sensitivity and 93.03% specificity, which is very high accuracy compared to the existing lung cancer screening method, so the present invention can effectively provide information on lung cancer diagnosis.

[Figure 1]

Control (Con)-445, Lung cancer patients (LC)-419, * P < 0.05, ** p < 0.005, *** p < 0.001

**Description**

[Technical field]

The present invention relates to a biomarker for diagnosing lung cancer and a method for providing artificial intelligence-based information for diagnosing lung cancer, and more particularly to a biomarker for diagnosing lung cancer comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), a biomarker composition for diagnosing lung cancer, and a method for providing artificial intelligence-based information for diagnosing lung cancer using the biomarker.

[Background art]

Cancer is a disease in which cells multiply indefinitely and disrupt normal cell function, most commonly in liver, lung, stomach, breast, colon, and ovarian cancers, but can occur in virtually any tissue.

Initially, cancer diagnosis was based on external changes in biological tissues caused by the growth of cancer cells, but in recent years, cancer diagnosis has been attempted using the detection of trace biomolecules present in the tissues or cells of living organisms, such as blood, glycol-chains, and DNA. However, the most commonly used methods of cancer diagnosis are tissue samples obtained through biopsy or imaging.

Among them, biopsies are painful for the patient, expensive, and require a long time to diagnose. In addition, if the patient has actual cancer, there is a risk that the biopsy process may cause the cancer to metastasize, and if the biopsy cannot obtain a tissue sample, the disease cannot be diagnosed until the suspected tissue is surgically removed.

Lung cancer, in particular, is one of the most lethal cancers in the world, and current diagnosis of lung cancer relies heavily on imaging methods (X-ray, CT, MRI, etc.). However, more than half of lung cancer patients are already inoperable at the time of diagnosis, and even if surgery is deemed feasible, many of them cannot be completely resected. Therefore, early diagnosis and treatment of lung cancer is of utmost importance in order to increase the cure rate of lung cancer, but lung cancer has limited markers useful for diagnosis, making such diagnosis difficult. Therefore, it is necessary to find cancer-specific markers present in biological samples and develop methods to diagnose cancer with high accuracy and precision using these markers.

In recent years, methods using artificial intelligence have been studied for more accurate cancer diagnosis. Machine learning or deep learning is mainly used for analysis using artificial intelligence (AI), and various studies are being conducted to utilize AI in the bio field (e.g. Korean Publication Patent No. 10-2014-0002149, Korean Registered Patent No. 10-2268963).

[Disclosure]

[Technical Problem]

Accordingly, the present inventors have made a diligent effort to screen for markers that can more accurately diagnose lung cancer. As a result, we have selected a set of biomarkers comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), and found that the expression levels of these biomarkers showed different patterns in patients and healthy controls. In addition, the quantitative values of 10 biomarkers were analyzed using an artificial intelligence-based algorithm, and the diagnostic ability of lung cancer was improved, and the invention was completed.

Accordingly, an object of the present invention is to provide a biomarker composition for diagnosing lung cancer.

Another object of the present invention is to provide a composition for diagnosing lung cancer comprising an agent (substance) that measures the expression level of the biomarker(s) and a diagnostic kit for lung cancer using the same.

Another object of the present invention is to provide a method for providing artificial intelligence-based information for diagnosing lung cancer using the biomarker(s).

[Technical solution]

To fulfill the above purposes, the present invention provides a biomarker composition for diagnosing lung cancer comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).

In a preferred embodiment of the present invention, the biomarker may be extracted from blood.

In another preferred embodiment of the present invention, blood may be whole blood, plasma, or serum.

To fulfill other purposes, the present invention provides a composition for diagnosing lung cancer, comprising an agent for measuring a level in blood of the biomarker composition for diagnosing lung cancer.

In a preferred embodiment of the present invention, the agent for measuring the level of the biomarker composition may be a mass spectrometry preparation.

The present invention also provides a kit for diagnosing lung cancer comprising an agent for measuring a level in blood of the biomarker composition for diagnosing lung cancer.

To fulfill another purpose, the present invention provides a method for providing artificial intelligence-based information for diagnosing lung cancer comprising:

(a) the step of measuring expression levels of biomarkers for diagnosing lung cancer from the blood of a test subject, wherein the biomarkers comprise kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(b) the step of applying the expression levels of the biomarkers to a machine learning algorithm model.

In a preferred embodiment of the present invention, the blood in step (a) may be whole blood, plasma, or serum.

In another preferred embodiment of the present invention, the level(s) of the biomarker(s) in step (a) above may be obtained by mass spectrometry of the whole blood, plasma or serum sample by mass peak area, more specifically by liquid chromatography-mass spectrometry (LC-MS), wherein the mass spectrometer may be any one of triple TOF, triple quadrupole and MALDI TOF capable of quantitative measurement.

In another preferred embodiment of the present invention, in the step (b) above of applying to the algorithmic model, the level of the biomarker in the blood of the test subject may be input to the algorithmic model to output as an output whether or not the test subject has lung cancer.

In another preferred embodiment of the present invention, the algorithmic model of step (b) above comprises or is derived from:

(i) measuring levels of the biomarker for diagnosing lung cancer from blood of a group of lung cancer patient and a group of normal control, wherein the biomarker comprises kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(ii) training the levels of the biomarker with a machine learning algorithm to generate a lung cancer incidence prediction model.

In another preferred embodiment of the present invention, the artificial intelligence may be machine Learning or deep Learning, and more specifically, the algorithm in step (b) above may be any one of a linear or nonlinear classification algorithm selected from the group consisting of a k-nearest neighbor algorithm; a logistic regression algorithm; a discriminant analysis algorithm; a partial least squares discriminant analysis algorithm; a support vector machine algorithm; a decision tree algorithm; a decision tree ensemble algorithm; and a neural network algorithm.

In another preferred embodiment of the present invention, where the algorithm is a support vector machine algorithm, it may be represented by the kernel function of Equation 1 below.

[Equation 1]

$$K(x_i, x_j) = \exp\left(-\frac{\|x_i - x_j\|^2}{2\sigma^2}\right)$$

**X:** Measured blood level of a biomarker composition for lung cancer diagnosis
σ: parameter for the flexibility (curvature) of the decision boundary

The present invention also provides an artificial intelligence-based lung cancer diagnosis prediction device comprising: a measurement part for measuring a biomarker level for diagnosing lung cancer in the blood of a test subject; and a cancer diagnosis part for inputting the biomarker level into a trained artificial intelligence algorithm to determine whether the test subject has occurred lung cancer.

[Advantageous Effects]

In this invention, 10 biomarkers that can diagnose lung cancer more accurately were selected, and an artificial intelligence-based algorithm for diagnosing lung cancer was established using them. The early screening ability of lung cancer using the algorithm developed in this invention was 90.21% sensitivity and 93.03% specificity, which was very high compared to the existing lung cancer screening method, so this invention can effectively provide information on lung cancer diagnosis.

[Description of Drawings]

FIG. 1 shows the levels of kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), in the blood of normal controls (Con) and lung cancer patients (LC).
Figure 2 shows the differential expression patterns of different biomarker types in the blood of lung cancer patients.

[Mode of the Invention]

The present invention will now be described in detail.

**Biomarker composition for diagnosing lung cancer**

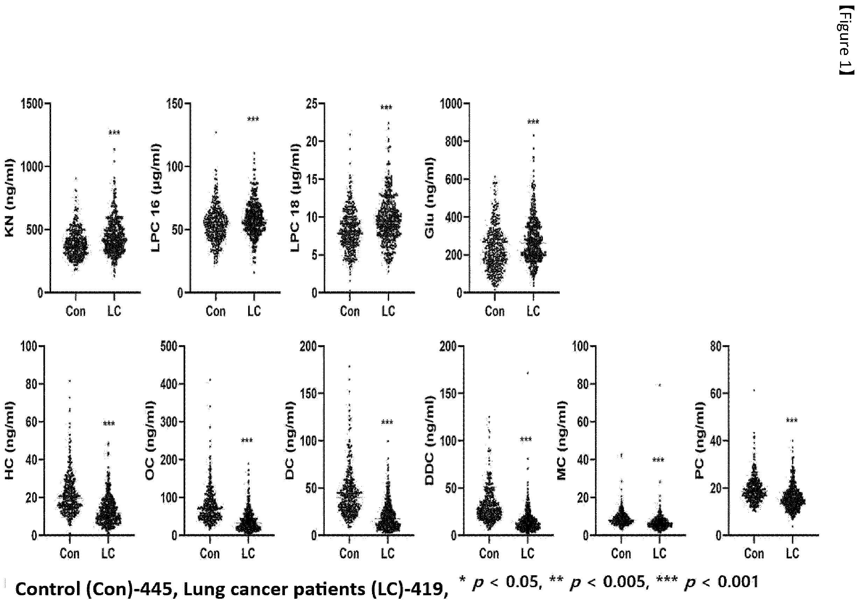

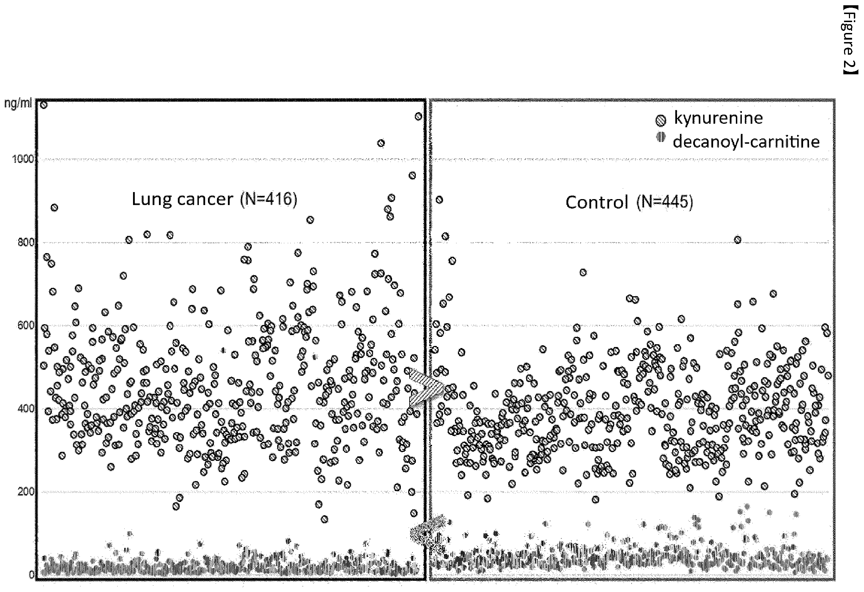

In one aspect, the present invention relates to a biomarker composition for diagnosing lung cancer comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).
In the present invention, the biomarker can be derived from blood, wherein the blood may be whole blood, plasma, or serum.
As used in the present invention, the term "diagnosis" refers to the identification of the presence or characterization of a pathological condition. For the purposes of the present invention, diagnosis can be the identification of the presence or absence of lung cancer.
As used in the present invention, the term "diagnostic biomarker" refers to an organic biomolecule, such as a polypeptide, nucleic acid (e.g., mRNA, etc.), lipid, glycolipid, glycoprotein, sugar (monosaccharide, disaccharide, oligosaccharide, etc.), or the like, that shows a significant increase or significant decrease in a group of lung cancer patients compared to a group of normal controls, and is preferably a biomarker composition for diagnosing lung cancer.
In a specific embodiment of the present invention, blood from a normal control group (Con) and a lung cancer (LC) patient group was obtained, and the concentrations (levels) of KN, LPC16, LPC18, Glu, HC, OC, DC, DDC, MC, and PC in the blood were measured. It was confirmed that the concentrations of the biomarkers of the present invention in the blood were significantly different from the quantitative values of the lung cancer patients and the normal controls, and it was confirmed that the concentrations of KN, LPC16, LPC18 and Glu metabolites were increased and the concentrations of HC, OC, DC, DDC, MC and PC metabolites were decreased in the blood of the lung cancer patients compared to the normal controls (FIG. 1 and FIG. 2).

**Composition for diagnosing lung cancer**

In another aspect, the present invention relates to a composition for diagnosing lung cancer comprising an agent for measuring a level in blood of the biomarker composition for diagnosing lung cancer of the present invention.
Specific details of the composition for diagnosing lung cancer according to the present invention can refer to "Biomarker composition for diagnosing lung cancer" above.
The biomarkers of the present invention may be metabolites, and the agent for measuring the level(s) of the biomarker composition may be an agent for mass spectrometry.
The agent for mass spectrometry means an agent capable of analyzing the mass of a marker in whole blood, plasma, or serum, and more specifically, an agent capable of performing liquid chromatography-mass spectrometry (LC-MS).

**Kit for diagnosing lung cancer**

In another aspect, the present invention relates to a kit for diagnosing lung cancer comprising an agent for measuring a level in blood of the biomarker composition for diagnosing lung cancer of the present invention.

Specific details of the composition for diagnosing lung cancer according to the present invention can refer to "Biomarker composition for diagnosing lung cancer" above.

The kits can be prepared by conventional methods known in the art. The kits may include, for example, antibodies in lyophilized form, buffers, stabilizers, inactive proteins, and the like.

**Method of providing information for diagnosing lung cancer using artificial intelligence-based algorithm**

In another aspect, the present invention relates to a method for providing artificial intelligence-based information for diagnosing lung cancer comprising:

(a) the step of measuring levels of biomarkers for diagnosing lung cancer from the blood of a test subject, wherein the biomarkers comprise kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(b) the step of applying the expression levels of the biomarkers to a machine learning algorithm model.

In the present invention, the blood in step (a) may be whole blood, plasma, or serum.

In the present invention, the level (concentration) of the biomarker in step (a) above may be obtained by mass spectrometric analysis of the whole blood, plasma or serum sample by mass peak area, more specifically by liquid chromatography-mass spectrometry (LC-MS), wherein the mass spectrometer may be any one of triple TOF, triple quadrupole and MALDI TOF capable of quantitative measurement.

In the present invention, the step (b) of applying to the algorithmic model may include inputting the level of the biomarker in the blood of the test subject into the algorithmic model to output as an output whether the test subject has lung cancer.

In the present invention, the algorithmic model in step (b) above may comprise,

(i) measuring levels of the biomarker for diagnosing lung cancer, comprising kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC), from blood of a group of lung cancer patients and a group of normal controls; and
(ii) training the levels of the biomarker with a machine learning algorithm to generate a lung cancer incidence prediction model.

In the present invention, the artificial intelligence may be machine learning or deep learning, and more specifically, the algorithm in step (b) above may be any one of linear or nonlinear classification algorithms selected from the group consisting of k-nearest neighbor algorithms; logistic regression algorithms; discriminant analysis algorithms; partial least squares discriminant analysis algorithms; support vector machine algorithms; decision tree algorithms; decision tree ensemble algorithms; and neural network algorithms.

In the present invention, where the algorithm is a support vector machine algorithm, it may be represented by the kernel function of Equation 1 below.

[Equation 1]

$$K(x_i, x_j) = \exp\left(-\frac{\|x_i - x_j\|^2}{2\sigma^2}\right)$$

X: Measured blood level of a biomarker composition for lung cancer diagnosis
σ: parameter for the flexibility (curvature) of the decision boundary

In a specific embodiment of the present invention, a prediction model was developed using the quantitative values of 10 biomarkers of the present invention measured in the blood of lung cancer patients and normal controls, and the algorithm was a support vector machine using a radial basis function as a kernel. The developed prediction model was used to check the early diagnostic ability of lung cancer, and it was found to have a sensitivity of 90.21% and a specificity of 93.03%.

In other words, it was confirmed that the AI-based lung cancer diagnosis method using the 10 biomarkers of the present invention has a very high accuracy compared to other existing diagnostic methods.

In another aspect, the present invention provides an artificial intelligence-based lung cancer diagnosis prediction device comprising: a measurement part for measuring biomarker levels for diagnosing lung cancer in the blood of a test subject, wherein the biomarkers comprise kynurenine (KN), lysophosphatidylcholine 16:0 (LPC16), lysophosphatidylcholine 18:0 (LPC18), lysophosphatidylcholine 18:0, LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and a cancer diagnosis part for inputting the biomarker level into a trained artificial intelligence algorithm to determine whether the test subject has occurred lung cancer.

The present invention will now be described in more detail with reference to the following examples.

These embodiments are intended solely to illustrate the invention, and it will be apparent to one of ordinary skill in the art that the scope of the invention is not to be construed as limited by these embodiments.

## Example 1: Measurement of biomarker concentrations in the blood of lung cancer patients and healthy controls

### 1-1: Sample preparation

To determine whether the biomarker of the present invention can diagnose lung cancer, a total of 864 patients were screened, specifically, 445 normal controls and 419 lung cancer (LC) patients were screened and blood was collected through Seoul National University Bundang Hospital and Ajou University Hospital.

### 1-2: Measuring biomarker concentrations in blood

From the blood sample, plasma was separated and standard materials for each concentration required for the biomarker standard curves of kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC) were prepared.

20 μl of plasma and each standard material were vortexed with 400 μl of lipid extraction buffer (lipid extraction buffer; Abnova, Taiwan) and centrifuged (10,000g, 5 min, 4°C) after vortexing. After centrifugation, all supernatants were transferred to a new tube and dried using a concentrator for 12 to 16 hours. To the dried metabolome extract, 50 μl of 100% methanol with 0.1% formic acid (FA) was added, dissolved well using a vortex, and analyzed using liquid chromatography-mass spectrometry/mass spectrometry (LC-MS/MS).

The LC used was a Shimadzu LC 40 system and the MS was an AB Sciex Triple Quad 5500+ system. The MS was equipped with a turbo spray ion source. The analytes were separated within a BEH C18 column (1.7um, 2.1*50mm; Waters) on a Shimadzu LC 40 system. The solvent used was a two-step linear gradient (solvent A, 0.1% FA in water; solvent B, 0.1% FA in 100% acetonitrile; 5 to 55% solvent B 2.5 min, 55% solvent B 5.5 min, 55 to 95% solvent B 7.5 min, 95% solvent B 11 min, 95 to 5% solvent B 11.1 min, and 5% solvent B 14.5 min).

Mass spectrometry (MS/MS) was performed using multiple reaction monitoring (MRM) mode. The area of the mass peak with the same mass value in the mass spectrum at the same time period as the time when the metabolite corresponding to each biomarker passed through the liquid chromatography was calculated. A standard curve was constructed using the mass peaks of each biomarker standard and the mass peaks of each sample were plotted against the standard curve to determine the concentration of each biomarker.

As a result, as shown in Figures 1 and 2, the blood concentrations of the 10 biomarkers were found to be significantly different between the quantitative values of the lung cancer patients and those of the normal controls, with increased concentrations of KN, LPC16, LPC18, and Glu metabolites and decreased concentrations of HC, OC, DC, DDC, MC, and PC metabolites in the blood of the lung cancer patients compared to the normal controls.

## Example 2: Developing an AI-based algorithmic model for lung cancer diagnosis

In this invention, a support vector machine algorithm using a radial basis function as a kernel was applied to the quantitative values of 10 biomarkers to develop a prediction model to diagnose whether lung cancer has occurred.

We trained a lung cancer incidence prediction model using the kernel function represented by Equation 1 below and tuning the algorithm parameters.

[Equation 1]

$$K(x_i, x_j) = \exp\left(-\frac{\|x_i - x_j\|^2}{2\sigma^2}\right)$$

X: Measured blood level of a biomarker composition for lung cancer diagnosis
σ: parameter for the flexibility (curvature) of the decision boundary

The parameter σ in Equation 1 determines the extent of influence of a single training sample, and the other parameter, C, determines how much training samples are allowed to be misclassified. Since both parameters underfit or overfit the learning model depending on their values, the optimal parameters were selected through iterative cross-validation.

[Table 1]

| Using algorithm to confirm lung cancer diagnostic ability | | | |
|---|---|---|---|
| | | TRUE | |
| | | Lung cancer | Control | |
| predicted | Lung cancer | 378 | 31 | 92.42% PPV |
| | Control | 41 | 414 | 90.99% NPV |
| | | 90.21% Sensitivity | 93.03% Specificity | |

As a result of checking the ability to early diagnose lung cancer using the present developed prediction model, it was found that the sensitivity was 90.21%, specificity was 93.03%, positive predictive value (PPV) was 92.42%, and negative predictive value (NPV) was 90.99%, as shown in Table 1. In other words, it was confirmed that the AI-based lung cancer diagnosis method using 10 biomarkers of the present invention has a very high accuracy compared to other existing diagnostic methods.

In this invention, 10 biomarkers that can diagnose lung cancer more accurately were selected, and it was confirmed that the early screening ability of lung cancer using the algorithm developed using these biomarkers was 90.21% sensitivity and 93.03% specificity, which has a very high accuracy compared to the existing lung cancer screening method, so this invention can be effectively applied to diagnose lung cancer.

**Claims**

1. A biomarker composition for diagnosing lung cancer comprising kynurenine (KN), lysophosphatidylcholine 16:0 (LPC16), lysophosphatidylcholine 18:0 (LPC18), lysophosphatidylcholine 18:0, LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC).

2. The biomarker composition for diagnosing lung cancer of claim 1, wherein the biomarker is derived from blood.

3. The biomarker composition for diagnosing lung cancer of claim 1, wherein the blood is whole blood, plasma, or serum.

4. A composition for diagnosing lung cancer, comprising an agent for measuring a level in blood of the biomarker composition for diagnosing lung cancer of any one of claims 1 to 3.

5. The composition for diagnosing lung cancer of claim 4, wherein the agent for measuring the level of the biomarker composition is an agent for mass spectrometry.

6. A kit for diagnosing lung cancer, comprising an agent for measuring a level in blood of a biomarker composition for diagnosing lung cancer of any one of claims 1 to 3.

7. A method of providing information for diagnosing lung cancer using artificial intelligence, comprising:

(a) the step of measuring levels of biomarkers for diagnosing lung cancer from the blood of the test subject, wherein the biomarkers comprise kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(b) the step of applying the levels of the biomarkers to a machine learning algorithm model.

8. The method of providing information for diagnosing lung cancer using artificial intelligence of claim 7, wherein the blood in step (a) is whole blood, plasma, or serum.

9. The method of providing information for diagnosing lung cancer using artificial intelligence of claim 7, wherein the measuring levels of biomarkers in step (a) is obtained via liquid chromatography-mass spectrometry (LC-MS).

10. The method of providing information for diagnosing lung cancer using artificial intelligence of claim 7, wherein the applying the levels of the biomarkers to the algorithmic model in step (b) is to input levels of the biomarkers in the blood of a test subject into the algorithmic model and output as an output whether the test subject has lung cancer.

11. The method of providing information for diagnosing lung cancer using artificial intelligence of claim 7, wherein the algorithmic model in step (b) comprises:

(i) the step of measuring levels of biomarkers for diagnosing lung cancer from the blood of the test subject, wherein the biomarkers comprise kynurenine (KN), lysophosphatidylcholine 16:0 (LPC16), lysophosphatidylcholine 18:0 (LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(ii) training the levels of the biomarker with a machine learning algorithm to generate a lung cancer incidence prediction model.

12. The method of providing information for diagnosing lung cancer using artificial intelligence of claim 7, wherein the algorithm of step (b) is any one of a linear or nonlinear classification algorithm selected from the group consisting of a k-nearest neighbor algorithm; a logistic regression algorithm; a discriminant analysis; a partial least squares discriminant analysis; a support vector machine algorithm; a decision tree algorithm; a decision tree ensemble algorithm; and a neural network algorithm.

13. The method of providing information for diagnosing lung cancer using artificial intelligence of claim 7, wherein the algorithm of step (b) is a support vector machine algorithm, wherein the algorithm is represented by the kernel function of Equation 1 below:

[Equation 1]

$$K\left(x_i, x_j\right) = \exp\left(-\frac{\left\|x_i - x_j\right\|^2}{2\sigma^2}\right)$$

in Equation 1, x is a measured blood level of a biomarker composition for lung cancer diagnosis, and σ is a parameter for the flexibility (curvature) of the decision boundary.

14. An artificial intelligence-based lung cancer diagnosis prediction device comprising:

(i) a measurement part for measuring biomarker levels for diagnosing lung cancer in the blood of a test subject, wherein the biomarkers comprise kynurenine (KN), lysophosphatidylcholine 16:0 (LPC16), lysophosphatidylcholine 18:0 (LPC18), lysophosphatidylcholine 18:0, LPC18), glutamic acid (Glu), hexanoyl-L-carnitine (HC), octanoyl-L-carnitine (OC), decanoyl-L-carnitine (DC), dodecanoyl-L-carnitine (DDC), myristoyl-L-carnitine (MC) and palmitoyl-L-carnitine (PC); and
(ii) a cancer diagnosis part for inputting the biomarker level into a trained artificial intelligence algorithm to determine whether the test subject has occurred lung cancer.

【Figure 1】

【Figure 2】

## INTERNATIONAL SEARCH REPORT

| International application No. |
|---|
| **PCT/KR2023/006352** |

**A. CLASSIFICATION OF SUBJECT MATTER**

**G01N 33/487**(2006.01)i; **G01N 30/72**(2006.01)i; **G06N 3/08**(2006.01)i

According to International Patent Classification (IPC) or to both national classification and IPC

**B. FIELDS SEARCHED**

Minimum documentation searched (classification system followed by classification symbols)

G01N 33/487(2006.01); A61N 2/00(2006.01); A61N 2/06(2006.01); G01N 30/72(2006.01); G01N 30/86(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폐암(lung cancer), 바이오마커(biomarker), 인공지능(artificial intelligence), 키뉴레닌(kynurenine), 리소포스파티딜콜린 16:0(lysophosphatidylcholine 16:0), 리소포스파티딜콜린 18:0(lysophosphatidylcholine 18:0), 글루탐산(glutamic acid), 핵사노일-L-카르니틴(hexanoyl-L-carnitine), 옥타노일-L-카르니틴(octanoyl-L-carnitine), 데카노일-L-카르니틴(decanoyl-L-carnitine), 도데카노일-L-카르니틴(dodecanoyl-L-carnitine), 미리스토일-L-카르니틴(myristoyl-L-carnitine), 팔미토일-L-카르니틴(palmitoyl-L-carnitine)

**C. DOCUMENTS CONSIDERED TO BE RELEVANT**

| Category* | Citation of document, with indication, where appropriate, of the relevant passages | Relevant to claim No. |
|---|---|---|
| Y | XIE, Y. et al. Early lung cancer diagnostic biomarker discovery by machine learning methods. Translational oncology. 2021, vol. 14, no. 1, article no. 100907, inner pp. 1-10.<br>See abstract; pages 1-5; and figure 2A. | 1-14 |
| Y | KR 10-2020-0116410 A (NATIONAL CANCER CENTER) 12 October 2020 (2020-10-12)<br>See paragraphs [0028]-[0031] and [0035]-[0036]. | 1-14 |
| Y | NI, J. et al. Simultaneous determination of thirteen kinds of amino acid and eight kinds of acylcarnitine in human serum by LC–MS/MS and its application to measure the serum concentration of lung cancer patients. Biomedical Chromatography. 2016, vol. 30, no. 11, pp. 1796-1806.<br>See table 6. | 1-14 |
| Y | MORENO, P. et al. Metabolomic profiling of human lung tumor tissues–nucleotide metabolism as a candidate for therapeutic interventions and biomarkers. Molecular oncology. 2018, vol. 12, no. 10, pp. 1778-1796.<br>See figure 4. | 1-14 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 September 2023** | **04 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/006352**

**C. DOCUMENTS CONSIDERED TO BE RELEVANT**

| Category* | Citation of document, with indication, where appropriate, of the relevant passages | Relevant to claim No. |
|---|---|---|
| A | US 2022-0118269 A1 (UNIVERSITY OF IOWA RESEARCH FOUNDATION) 21 April 2022 (2022-04-21)<br>See entire document. | 1-14 |

Form PCT/ISA/210 (second sheet) (July 2022)

**INTERNATIONAL SEARCH REPORT**
Information on patent family members

International application No.

**PCT/KR2023/006352**

| Patent document cited in search report | | | Publication date (day/month/year) | Patent family member(s) | | | Publication date (day/month/year) |
|---|---|---|---|---|---|---|---|
| KR | 10-2020-0116410 | A | 12 October 2020 | KR | 10-2395558 | B1 | 10 May 2022 |
| US | 2022-0118269 | A1 | 21 April 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020140002149

- KR 102268963